# EUROPEAN PATENT APPLICATION

(11) **EP 4 372 380 A1**
(43) Date of publication of application: **22.05.2024**
(21) Application number: 22383112.4
(22) Date of filing: 17.11.2022
(51) Int. Cl.: G01N 33/574

(54) **MICROBIOTA-ASSOCIATED MARKERS OF HIGH-GRADE SQUAMOUS INTRAEPITHELIAL LESIONS (HSIL)**

(71) Applicant: Consejo Superior de Investigaciones Científicas (CSIC), 28006 Madrid (ES); Fundación Para La Investigación Biomédica Del Hospital Universitario Ramón Y Cajal, 28034 Madrid (ES)
(72) Inventor: SERRANO VILLAR, Sergio, 28034 Madrid (ES); FERRER MARTÍNEZ, Manuel, 28006 Madrid (ES)
(74) Representative: Hoffmann Eitle

(57) **Abstract**

The present invention relates to the field of cancer diagnosis. Specifically, it relates to an improved method for the screening, diagnosis and/or monitoring of precancerous anal lesions, in particular high-degree squamous intraepithelial lesions (HSIL), in a human subject.

## Description

### Technical field of the invention

The present invention relates to the field of cancer diagnosis. Specifically, it relates to an improved method for the screening, diagnosis and/or monitoring of precancerous anal lesions, in particular high-degree squamous intraepithelial lesions (HSIL), in a human subject.

### Background of the invention

Anal cancer, uncommon in the general population, is preceded by high-grade squamous intraepithelial lesions (HSILs). The risk of anal cancer is surprisingly high in people with HIV (PWH), especially among men who have sex with men (MSM) (85 to 300-fold) (Clifford, G. M. et al. A meta-analysis of anal cancer incidence by risk group: Toward a unified anal cancer risk scale. International Journal of Cancer 148, 1-11 (2020). Koroukian, S. M. et al. Excess cancer prevalence in men with HIV: A nationwide analysis of Medicaid data. Cancer 128, 1987-1995 (2022)), in whom screening and treating HSIL decreases the risk of anal cancer (Palefsky, J. M. et al. Treatment of Anal High-Grade Squamous Intraepithelial Lesions to Prevent Anal Cancer. New England Journal of Medicine 386, 2273-2282 (2022)). Because several studies have suggested a role for the microbiota as a relevant actor in the persistence and oncogenic transformation of human papillomavirus (HPV)-the underlying cause-, the anal bacterial communities offer an opportunity for the discovery of diagnostic biomarkers (Ilhan, Z. E. et al. Deciphering the complex interplay between microbiota, HPV, inflammation and cancer through cervicovaginal metabolic profiling. EBioMedicine 44, 675-690 (2019). Norenhag, J. et al. The vaginal microbiota, human papillomavirus and cervical dysplasia: a systematic review and network meta-analysis. BJOG: An International Journal of Obstetrics & Gynaecology 127, 171-180 (2020). Audirac-Chalifour, A. et al. Cervical Microbiome and Cytokine Profile at Various Stages of Cervical Cancer: A Pilot Study. PLoS One 11, e0153274 (2016). Ron, R. et al. Exploiting the Microbiota for the Diagnosis of Anal Precancerous Lesions in Men Who Have Sex With Men. J Infect Dis 224, 1247-1256 (2021)).

While the reasons why PWH and MSM exhibit an increased risk of anal cancer remains unclear, both factors determine microbiota changes, which appear to contribute to heightened inflammation and immune dysfunction (Noguera-Julian, M. et al. Gut Microbiota Linked to Sexual Preference and HIV Infection. EBioMedicine 5, 135-146 (2016). 9. Vujkovic-Cvijin, I. et al. HIV-associated gut dysbiosis is independent of sexual practice and correlates with noncommunicable diseases. Nature Communications 11, (2020)). In MSM with HIV, the authors of the present invention previously explored the anal microbiota and identified several candidate bacterial taxa related to HSIL(Serrano-Villar, S. et al. HIV, HPV, and microbiota: Partners in crime? Aids 31, 591-594 (2017)). In women, the cervical microbiota seems also implicated in the development of HSIL, where proinflammatory species would sustain immune disbalance and a pro-carcinogenic milieu. Besides the HPV and HIV fields, emerging evidence supports that intestinal bacterium might amplify or mitigate carcinogenesis (Dalal, N. et al. Gut microbiota-derived metabolites in CRC progression and causation. J Cancer Res Clin Oncol 147, 3141-3155 (2021). Fu, A. et al. Tumor-resident intracellular microbiota promotes metastatic colonization in breast cancer. Cell 1356-1372 (2022) doi:10.1016/j.cell.2022.02.027). While it will take time to understand the cause-effect relationship, i.e., whether the microbiota influences HPV oncogenic transformation or simply reacts as a consequence of HPV progression, these host-microbe interactions can be harnessed to discover diagnostic biomarkers.

At any rate, the discriminative ability of the reference screening tool-anal cytology- is poor (~50%) (Clarke, M. A. & Wentzensen, N. Strategies for Screening and Early Detection of Anal Cancers: A Narrative and Systematic Review and Meta-Analysis of Cytology, HPV Testing, and Other Biomarkers. Cancer Cytopathology 126, 447-460 (2018)), which leads to an excessive number of invasive procedures, avoidable patient discomfort, and adverse events, challenging the implementation of this screening strategy and undermining the prevention of anal cancer. Therefore, new diagnostic procedures are warranted to overcome the limitations of cytology-based screening of anal cancer. For the present invention, we investigated the composition and functions of the anal microbiota associated with HSIL and validated two biomarkers with an excellent discriminative capacity able to overperform the reference test.

### Brief description of the figures

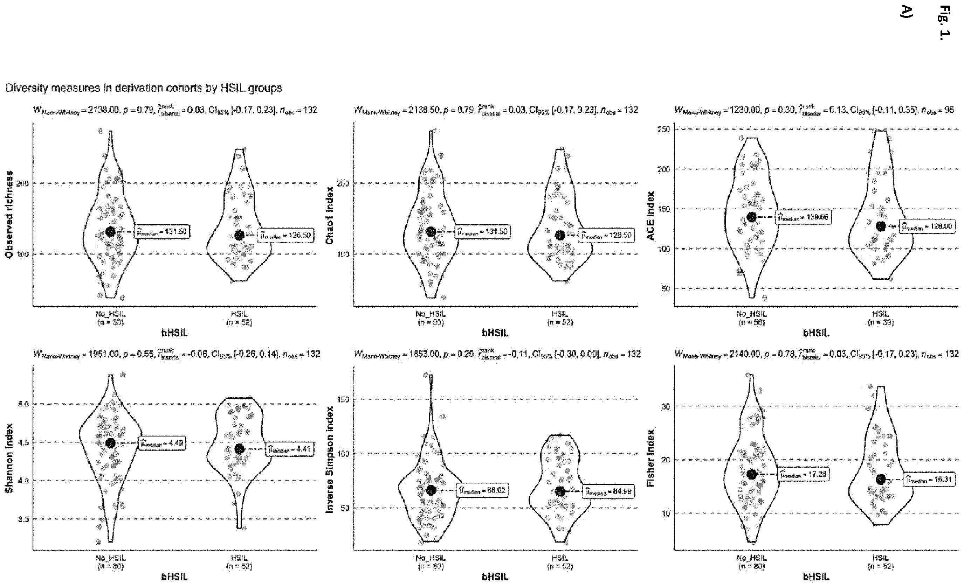

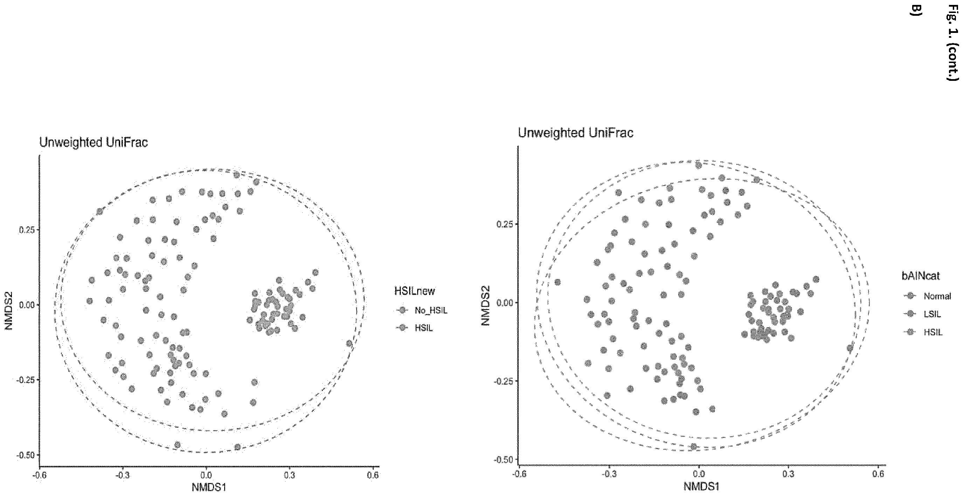

**Figure 1****.** a) Alpha diversity of the anal microbiota in the discovery cohort. b) NMDS analysis of beta diversity based on Unweighted Unifrac distances in participants in the discovery cohort. The NMDS analysis did not identify clusters based on the presence of HSIL (Permanova, P-value = 0.58 and 0.45). We appreciated a cluster of 42 subjects that compared to the rest of the individuals were less commonly smokers (19% vs. 39.5%), less frequently had a past history of anal condyloma (25.7% vs. 47%) and were more commonly recruited at one of the sites (95.2% vs. 72.3%), but none of these factors was statistically significant in a Permanova analysis adjusted for these variables.
**Figure 2****.** A. Distribution of the relative abundance at the phylum level of annotated peptides for the comparison HSIL vs. no HSIL.

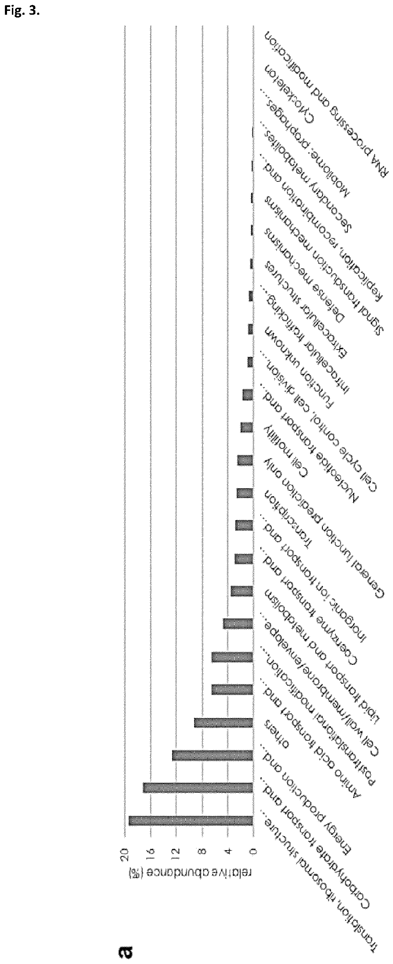

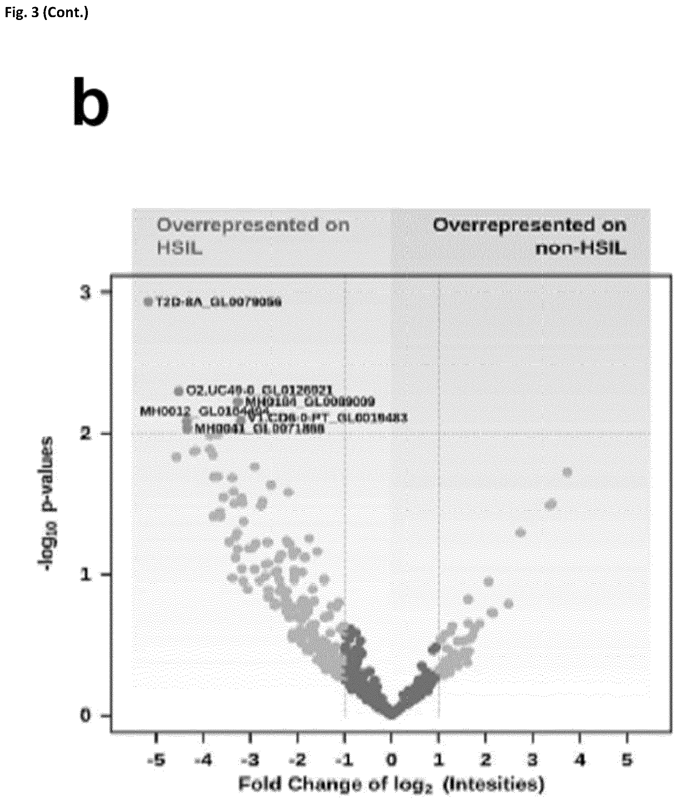

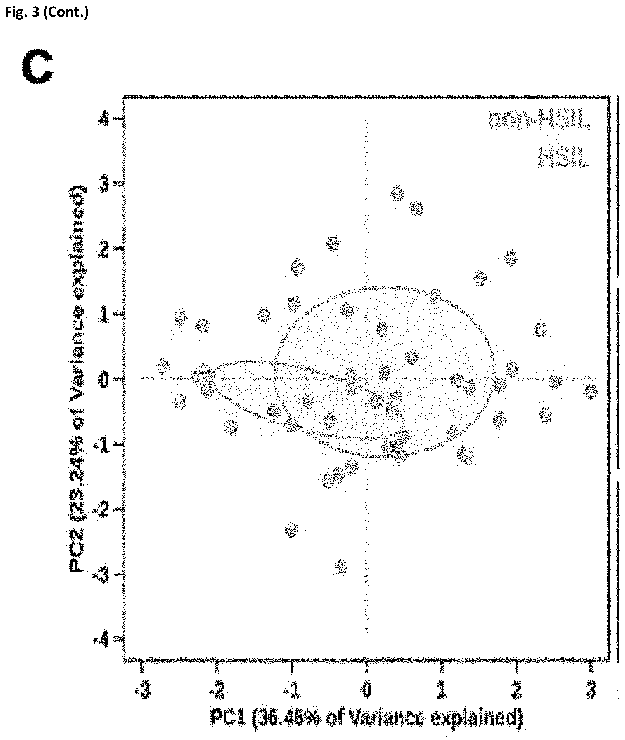

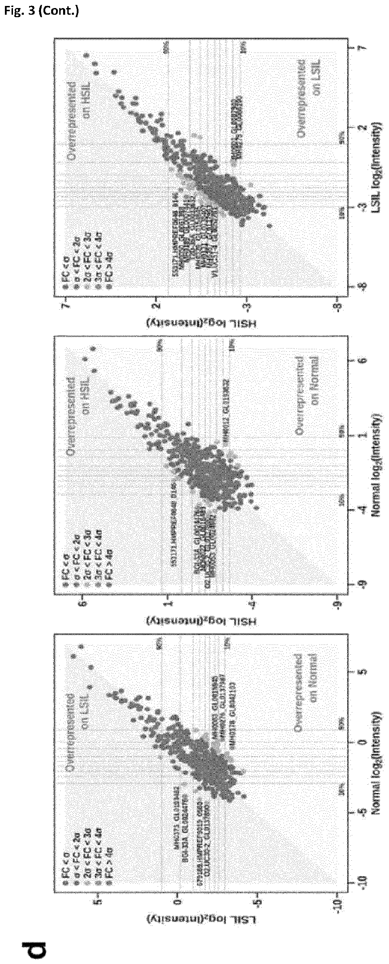

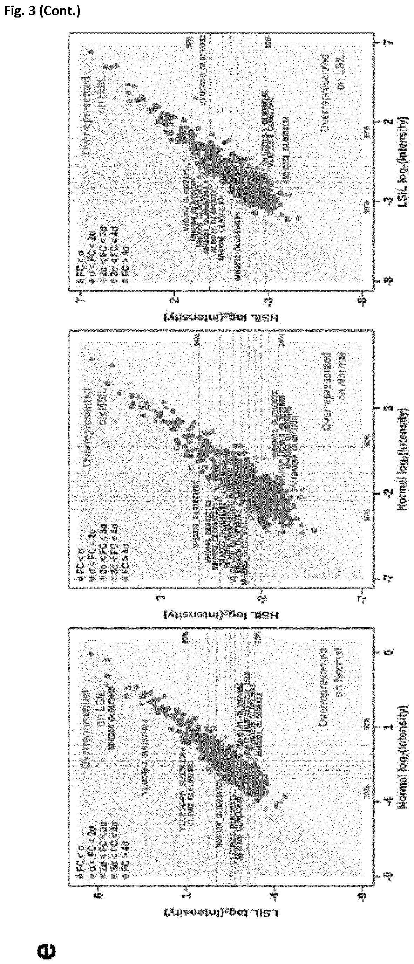

**Figure 3****.** a) Relative abundance of microbiome functions (COG categories). b) Volcano plot showing the relation among the Fold Change (FC) values of each protein with the log₁₀ of the p-values returned from the *moderate t-test* performed comparing each protein value between groups. Proteins with FC > 2 and moderate t-test p-values < 0.05 are shown on pink. c. PCA for proteins overrepresented in HSIL using the log2-transformed normalized ion intensities, explaining ~68% of the variance between PC1 and PC2. d) FC diagram showing the differential protein expression based on the histological HSIL, LSIL or normal information and e) for the same comparison's endpoint combining the cytological HSIL diagnosis not confirmed in biopsies. Proteins are highlighted according to the proportion of their absolute FC value to the standard deviation of all FC values. Proteins which FC > 2, 3, and 4σ have been highlighted in green, pink, and purple. The functional profiles were characterized in the 55 samples (13 in the HSIL group and 32 in the non-HSIL group) that were selected after discarding those with less than 500 proteins with an intensity >0.
**Figure 4****.** a) Pathway within the glycolysis and gluconeogenesis over-expressed in HSIL individuals. b) Connection of proteins found to be overexpressed in HSIL. The proteins overexpressed in HSIL are in blue, in black the proteins not overexpressed.
**Figure 5****.** Concentration of microbial succinyl-CoA in the derivation (upper panel or each subplot) and validation (lower panel of each subplot) cohorts in the compartison between HSIL vs. non-HSIL (a) and HSIL vs. LSIL vs. normal (b).

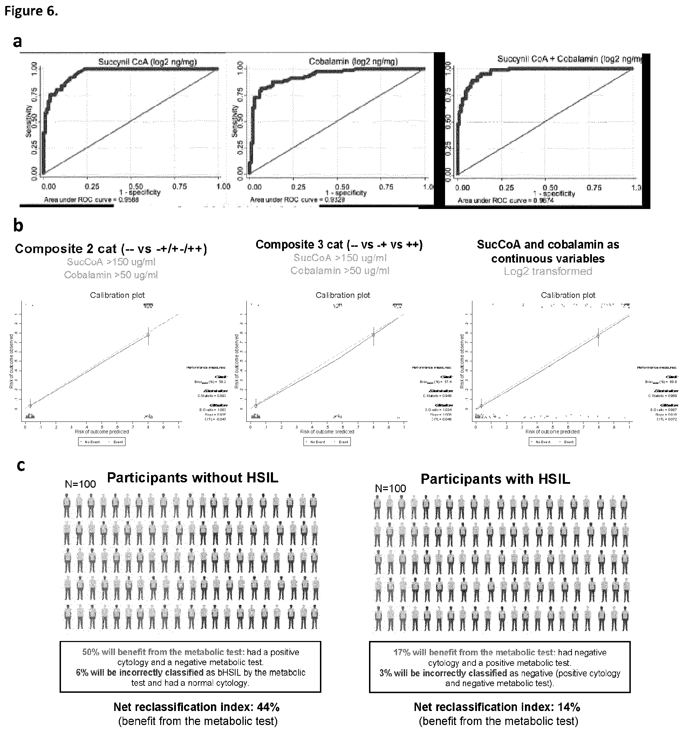

**Figure 6****.** a) Area under the receiver operating characteristic curve (AUC) estimates of the logistic regression models for the diagnosis of bHSIL in the discovery cohort, including either the bacterial markers alone or in combination. b) Calibration plot for observed and predicted estimates. Abbreviations: CITL, calibration in the large; E, expected; O, observed. c) Net reclassification indexes in participants without HSIL (left plot) or with HSIL (right plot). Left: 44% of individuals without HSIL benefited from the metabolic test (net reclassification index, (49-6)/98). Right: We also found a benefit, although smaller, among the 77 subjects with HSIL, of whom 13 had had negative cytology and then, were false-negative cytologic results, but had a positive metabolic test. In return, 2 cases were incorrectly classified as negative by the metabolic test despite abnormal cytology. Globally, 14% of individuals with HSIL benefited from the metabolic test (net reclassification index, (13-2)/77).
**Figure 7****.** Rho Spearman's correlation for cobalamin and Syccynil CoA levels. The regression line was adjusted using a local polynomial regression fitting (*loess* function).
**Figure 8****.** Summary of sample preparation.

### Description - Definitions

The terms "subject", or "individual‴ are used herein interchangeably to refer to all the animals classified as mammals and includes but is not limited to domestic and farm animals, primates and humans, for example, human beings, non-human primates, cows, horses, pigs, sheep, goats, dogs, cats, or rodents. Preferably, the "subject", or "individual" is a male or female human being of any age or race. More preferably, the term "subject", or "individual" is a human male, more preferably a human male with HIV (PWH), even more preferably a human male with HIV who has sex with men.

The term "sensitivity" as used herein refers to the proportion of subjects who have the target condition (reference standard positive) and give positive test results (TP/ (TP + FN)). It shows how good the test is at detecting a disease. Sensitivity ("sens") may be within the range of 0 (0%) < sens < 1 (100%) and ideally, the number of false negatives equaling zero or close to equaling zero and sensitivity equaling one (100%) or close to equaling one (100%).

The term "specificity" as used herein refers to the proportion of subjects without the target condition (reference standard negative) and give negative test results (TN/ (TN + FP)). It shows how good the test is at identifying normal (negative) condition. Specificity ("spec") may be within the range of 0 (0%) < spec < 1 (100%) and ideally, the number of false positives equaling zero or close to equaling zero and specificity equaling one (100%) or close to equaling one (100%).

The term "accuracy" as used herein refers to the proportion of true results, either true positive or true negative, in a population. It measures the degree of veracity of a screening test on a condition, i.e., how correct is the determination and exclusion of a given condition (TN + TP)/(TN+TP+FN+FP). Accuracy ("acc") may be within the range of 0 (0%) < acc < 1 (100%) and ideally, the number of false positives and false negative equaling zero or close to equaling zero and accuracy equaling one (100%) or close to equaling one (100%).

The term "therapeutically effective amount" as used herein refers to an amount that is effective, upon single or multiple dose administration to a subject (such as a human patient) in the prophylactic or therapeutic treatment of a disease, disorder or pathological condition.

### Description of embodiments

In the present invention, we confront the urgent need to identify and validate objective biomarkers to better stratify the risk of anal cancer. Here, we aimed to improve the diagnostic screening of HSIL by the detection of microbiome-associated biomarkers in anal swabs. Our analyses indicate that HSIL-associated bacteria overexpress proteins that converge in the production of succinyl-CoA and cobalamin, which levels were consistently increased in subjects with HSIL. The combination of both biomarkers clearly overperformed the reference test, the anal cytology, drastically reducing the number of false positive results. Who to screen for HSIL by HRA or how often to repeat the screening are important aspects of the clinical decision-making process for which we face many knowledge gaps, despite intense research on HPV biomarkers during the past decade. Extrapolating the data from cervical cancers, several studies have assessed a number of biomarkers in anal swabs including high-risk HPV genotyping testing, expression of E6/E7 oncogenes, the aberrant co-expression of Ki67, a marker of cell proliferation, and p16, a marker of cell-cycle arrest p16, or the extent of DNA methylation, an epigenetic marker of oncogenesis. However, these biomarkers have failed to consistently improve the discrimination ability compared to the anal cytology, and new biomarkers to correctly classify patients are warranted.

Recent studies have demonstrated that dysbiotic microbiota is involved in HPV persistence, progression of cervical neoplasia, and genital inflammation. In women, the interaction between the microbiota and HPV acquisition, and persistence have focused on the potential cause-effect relationship between the microbiota and HPV pathogenesis. A highly diverse vaginal microbiota dominated by non-Lactobacilli species or *Lactobacillus iners* determines a three to five times higher risk of any prevalent HPV and two to three times higher risk for high-risk HPV and dysplasia or cervical cancer compared with women with a vaginal microbiota dominated by *Lactobacillus crispatus.* This microbiota pattern-decreased diversity and *Lactobacillus spp.* abundance- was also related to HPV persistence in a cohort of twin premenopausal women HPV-discordant, suggesting a role of the microbiota besides genetic determinants on the HPV infection, and to regression of cervical dysplasia. Bacteria including Gardnerella, Prevotella, Megasphoera, and Atopobium genus consistently correlate with persistent cervicovaginal HPV infection. Therefore, this microbiota pattern is expected to have functional implications in oncogenesis, such as the depletion implicated in tempering genital inflammation.

In men, most studies have focused on characterizing the microbiota compositional patterns associated with anal HPV rather than on understanding the mechanisms influencing HPV persistence and oncogenesis. In a cross-sectional study, Nowak et al. found that MSM with HIV who have HPV-16 exhibit a microbiota enriched with members of the Fusobacteria phylum, but the association with anal precancerous lesions could not be studied (Wang, J. et al. High-Risk HPV16 E6 Activates the cGMP/PKG Pathway Through Glycosyltransferase ST6GAL1 in Cervical Cancer Cells. Front Oncol 11, (2021)). In an exploratory study in 42 MSM HIV-infected undergoing screening for anal HSIL, we found in anal biopsies that *Catenibacterium spp.* was enriched in subjects with HSIL. In a subsequent study including 128 MSM with and without HIV infection, we found in anal swabs that *Aloprevotella spp.* and Ruminococaceae family were enriched in subjects with HSIL. While *Prevotella melanonigenica* and *Sneathia sanguinegens* were depleted (Ron, R. et al. Exploiting the Microbiota for the Diagnosis of Anal Precancerous Lesions in Men Who Have Sex With Men. J Infect Dis 224, 1247-1256 (2021)). In contrast, in the present invention and as shown in the examples, the HSIL-associated pattern included enrichment for *Prevotella copri* and depletion of *Streptococcus periodonticum, Dialister succinatiphilus, Prevotella stercorea, Sneathia sanguinegens, Fusobacterium goniadiaformans, and Anaerovibrio lipolyticus.* **Several factors could explain the differences, being the HSIL definition the most important. In our previous study, HSIL cases included current and prior diagnoses during the past year, but approximately a 30% of HSIL cases resolve spontaneously after one year. Here, we used a more accurate case definition, in which our outcome variable was defined as current HSIL defined as histologically proven HSIL, which is the gold standard definition** (Barroso LF, et al. Clin Infect Dis 13;74(Suppl_2), S179-S192 (2022)). Second, 16S sequencing methods cannot accurately identify the species level, making the differences at this level less relevant. Last, our previous study was performed in a different setting, the sample size was smaller, and lacked an external validation cohort.

Metabolic reprogramming is a cornerstone of oncogenesis. In the case of HPV, infected cells alter their metabolism through the regulation of genes involved in the biogenesis of ribosomes and mitochondria, as well as genes involved in the metabolism of glucose, nucleotides, and glutamine. But if we want to harness these host-microbiota interactions, we need to understand the metabolic pathways by which the microbiota might influence the HPV oncogenic transformation of epithelial cells. Our study further supports the notion that **more relevant than the microbiota composition are its metabolic consequences.** We found several pathways at the crossroads between cancer, immune response, and bacterial metabolism illustrating how the anal microbiota influence HPV persistence and oncogenesis. For example, enolase expression was increased in the anal microbiota of subjects with HSIL. Enolase is a glycolytic enzyme, which catalyzes the conversion of 2-phosphoglycerate to phosphoenolpyruvate. An increased expression of this enzyme is commonly observed in cancer and relates to the Warburg effect, an adaptive response of tumor cells to hypoxia that allows them to maintain their metabolic fitness. But immune cells, cancer cells, or bacteria can also express enolase in the cell surface or the extracellular space, either within exosomes or as a soluble protein produced. Outside the cell, enolase activates the serin protease plasmin (PLA), a zymogen that results in the induction of collagenase activity, degrading fibrin and other matrix proteins. As a result, enolasedependent PLA formation allows immune cells, cancer cells, and pathogens, to invade tissue, leading to inflammation, metastasis, or infection.

Protein kinase G1 (PKG1), represented another relevant protein overexpressed in the microbiota of subjects with HSIL. PKG1 is the central effector for cGMP signaling and is highly conserved in Archaea, Bacteria, and eukaryotes, as a key enzyme for carbohydrate degradation via glycolysis. PKG1 is also overexpressed in many solid cancers, in which it is implicated in cell invasion, angiogenesis, and tumor growth During infection with high-risk HPV genotypes, HPV promotes glycosylation of cervical cells via ST6GAL1 induction, which correlates with overexpression of the oncogene E6, a major determinant of malignant transformation via downstream activation of the cGMP/PKG signaling pathway. Suppression of the cGMP/PKG pathway in cervical cancer cell lines blocks cancer cell growth, further suggesting that PKG1 represents a relevant molecular mechanism of HPV-related oncogenesis, and hence, a potential diagnostic marker. Another virus establishing a chronic infection, HIV-1, damages epithelial cell junctions by triggering PKG1-mediated phosphorylation of stathmin, disrupting the microtubule cytoskeleton, which represents another mechanism by which some bacteria could facilitate the progression of HPV-associated dysplasia.

Our analyses also pointed to the metabolism of branched-chain amino acids (e.g. valine, isoleucine) and cobalamin biosynthesis and transport as significant pathways in patients with HSIL. Cobalamin is required for the conversion of valine- and isoleucine-derived methylmalonic acid to succinyl-CoA, which can subsequently be oxidized in the TCA cycle or serve as a precursor for gluconeogenesis. Many solid tumors including melanoma, nasopharyngeal carcinoma, breast cancer, and cervical cancer, boost their branched-chain amino acid metabolism to support their metabolic demand, promote cell proliferation, and inhibit their apoptosis. Our findings argue that in the anal microbiota, distinct metabolic pathways necessary for bacterial energy production and motility, also influence host functions that impact HPV oncogenesis.

The major strengths of our study include the clinical relevance, since we focused on a leading cancer in PWH for which we need better screening tools; the novelty, since we define two new biomarkers of HSIL in the anal microbiota; and the robustness of our findings, since we used an experimental validation of the proteomic data by targeted metabolite analysis, and we validated the identified markers in an external cohort.

In conclusion, we found that cobalamin and succinyl-CoA are dramatically increased in the anal microbiota of subjects with precancerous lesions, exhibiting an excellent discriminative ability to identify subjects with HSIL and offering a new method to improve anal cancer prevention. When we compared their diagnostic accuracy to that of the reference test, the anal cytology, the combination of both metabolites was clearly superior. Therefore, cobalamin and succinyl-CoA concentrations, which might be readily measured in most clinical settings, can overcome the low specificity of anal cytology, a major hurdle of anal cancer screening.

Therefore, a first aspect of the invention refers to an in *vitro* or ex vivo use of the intracellular concentration of succinyl CoA and/or cobalamin obtained from the microbial cells of a biological sample comprising anal microbial cells, obtained or isolated from the anal canal of a subject in need thereof, preferably a human subject, as microbiota-associated markers of high-grade squamous intraepithelial lesions (HSIL).

It is noted that, in the context of the present invention, "high-grade squamous intraepithelial lesions" (HSIL) is understood as an area of abnormal cells that forms on the surface of the anal epithelium. HSIL is a squamous cell abnormality associated with human papillomavirus (HPV) and is found when the anal cytology samples or biopsies are examined by optic microscopy. HSIL encompasses the entities previously termed anal intraepithelial neoplasia (AIN)2, AIN3, moderate and severe dysplasia and carcinoma in situ (https://www.ncbi.nlm.nih.gov/books/NBK430728/).

Histologic criteria for HSIL exceeds the extent and degree of nuclear atypia allowed for a diagnosis of "low-grade squamous intraepithelial lesions (LSIL)" and includes less maturation, a higher nuclear-tocytoplasmic ratio, decreased organization from the lower immature cell layers to the superficial mature layers (loss of polarity), a greater degree of nuclear pleomorphism, highly irregular nuclear contours, increased mitotic index and abnormal mitotic figures, especially within more superficial layers of the epithelium. CIN3 must have full thickness atypia. When faced with not-so-straightforward biopsies where the pathologist is debating between benign mimics of HSIL, such as immature metaplasia or atypical atrophy, utilizing the biomarker p16 may help distinguish them, as p16 shows intense and continuous staining in HSILs and suggests infection with a high-risk HPV type (REF).

As used herein, "anal canal" is understood as is the most terminal part of the lower GI tract/large intestine, which lies between the anal verge (anal orifice, anus) in the perineum below and the rectum above.

It is further noted that in the context of the present invention, a subject or individual is understood as any animal classified as mammal and includes but is not limited to domestic and farm animals, primates and humans, for example, human beings, non-human primates, cows, horses, pigs, sheep, goats, dogs, cats, or rodents. Preferably, the "subject", or "individual" is a male or female human being of any age or race. More preferably, the term "subject", or "individual" is a human male, more preferably a human male with HIV (PWH), also preferably a human male that has sex with men, even more preferably a human male with HIV who has sex with men.

As used herein, men that has sex with men (MSM) is understood as are male persons who engage in sexual activity with members of the same sex.

As used herein, a person with HIV (PWH) is understood as persons with confirmed HIV infection.

In a preferred embodiment of the first aspect of the invention, the biological sample is an anorectal epithelium sample, preferably an anorectal epithelium sample taken from a human subject.

In another preferred embodiment of the first aspect of the invention, the anorectal epithelium sample is collected or isolated from the subject by using any of the list consisting of cytobrushes with and without spatulas, cytopicks, cotton, Dacron, preferably a dacron-tipped swab, rayon, or a swab such as a nylon-flocked (NF)-swab. It is noted, that in the examples of the present invention, the examples show the use of the Anex Brush (Rovers Medical Devices, Netherlands) to obtain the anal samples.

In another preferred embodiment of the first aspect of the invention, the biological sample is a human biological sample, preferably obtained from a human male, preferably from a human adult male having HIV and/or that has sex with men.

A second aspect of the invention refers to a method for quantifying the intracellular concentration of succinyl CoA and/or cobalamin present in a biological sample, preferably a human biological sample, comprising anal microbial cells, obtained or isolated from the anal canal of a subject in need thereof, the method comprising:
a. Lysing the microbial cells of the biological sample to obtain the intracellular content of the cells; and
b. Quantifying the intracellular concentration of succinyl CoA and/or cobalamin from the intracellular content of the cells.

As used herein, the quantification of step b) is preferably carried out by a method selected from the list consisting of ELISA, colorimetric/fluorimetric, nephelometry or mass spectrometry analysis.

In a preferred embodiment of the second aspect of the invention, the biological sample comprising anal microbial cells is an anorectal epithelium sample, preferably collected or isolated from cytobrushes, spatulas, cytopicks, cotton, Dacron, preferably a dacron-tipped swab, rayon, or a swab such as a nylon-flocked (NF)-swab.

In another preferred embodiment of the second aspect of the invention, the anorectal epithelium sample is collected by a swab, preferably by a dacron-tipped swab.

In another preferred embodiment of the second aspect of the invention, the subject is understood as any animal classified as mammal and includes but is not limited to domestic and farm animals, primates and humans, for example, human beings, non-human primates, cows, horses, pigs, sheep, goats, dogs, cats, or rodents. Preferably, the subject is a male or female human being of any age or race. More preferably, the subject is a human male, more preferably a human male with HIV (PWH), also preferably a human male that has sex with men, even more preferably a human male with HIV who has sex with men.

In another preferred embodiment of the second aspect of the invention, the intracellular content of step a) is obtained by separating the lysed cell content by centrifugation to obtain a supernatant comprising the proteins involved in the metabolism of succinyl CoA and/or cobalamin, and those metabolites themselves.

A third aspect of the invention refers to a method for determining a risk of a subject having precancerous anal lesions consisting of high-degree squamous intraepithelial lesions (HSIL), the method comprising:
a. quantifying the intracellular concentration of the microbiota-associated markers succinyl CoA and/or cobalamin present in a biological sample in accordance with the second aspect of the invention or in any of its preferred embodiments; and
b. classifying the biological sample of the subject as presenting an increased or reduced risk of having precancerous anal lesions consisting of high-degree squamous intraepithelial lesions (HSIL) based on the concentration obtained in a) for each of the microbiota-associated markers or its combination.

In a preferred embodiment of the third aspect of the invention, step b) classifies the subject by comparing the concentration obtained in a) for one or two of the microbiota-associated markers, or a combined score of the same, in the subject's biological sample, with one or more reference values; wherein said comparison between the concentration obtained in a) for the one or two of the microbiota-associated markers, or a combined score of the same, with said one or more reference values provides an indication of a risk of the subject having precancerous anal lesions consisting of high-degree squamous intraepithelial lesions (HSIL). Thereby, a subject is classified as a subject at risk of having precancerous anal lesions consisting of high-degree squamous intraepithelial lesions (HSIL) or not depending on said comparison. Preferably, said one or more reference values are about 50 ng of succinyl-CoA/mg of total protein present in the intracellular content of the biological sample and/or about 150 ng cobalamin / mg of total protein present in the intracellular content of the biological sample. More preferably, the subject is classified as a subject at risk of having precancerous anal lesions consisting of high-degree squamous intraepithelial lesions (HSIL) if:
- the intracellular concentration of succinyl-CoA obtained in a) is greater than about 50 ng succinyl-CoA /mg of total protein present in the biological sample; and/or
- the intracellular concentration of cobalamin obtained in a) is greater than about 150 ng of cobalamin /mg of total protein present in the biological sample.

More preferably, the subject is classified as a subject at risk of having precancerous anal lesions consisting of high-degree squamous intraepithelial lesions (HSIL) if:
- the intracellular content of succinyl-CoA obtained in a) is greater than about 50 ng succinyl-CoA/mg of total protein present in the biological sample; and
- the intracellular content of cobalamin obtained in a) is greater than about 150 ng of cobalamin /mg of total protein present in the biological sample.

It is noted that the term "about" is understood as ±30%, ±20%, ±10%, ±5% or ±1% of the indicated amount. Therefore, a ±10% of 150 shall be understood as any number between 135 and 165 including both extremes of the range, that is, the inferior (135) and the upper (165) extremes of the range.

As used herein, the total protein of the intracellular content present in the biological sample is preferably determined by using the PierceTM 660 nm Protein Assay Reagent, following the recommendation by the supplier (Thermo Scientific, Rockford, USA).

The method according to any of the second or third aspects of the invention, wherein said method further comprises storing the method results.

A fourth aspect of the invention refers to a computer-implemented method for determining a risk of a subject having precancerous anal lesions consisting of high-degree squamous intraepithelial lesions (HSIL), comprising the following steps:
(a) receiving or storing or having access to data comprising the intracellular concentration of succinyl CoA and/or cobalamin obtained from the microbial cells of a biological sample comprising anal microbial cells;
(b) Calculating a score value based on the comparison of the intracellular concentration of succinyl CoA and/or cobalamin, or a combined score of the same, obtained from the microbial cells of a biological sample comprising anal microbial cells received or stored in accordance with step a) with one or more reference values; and
(c) Returning as a result a risk or an indication of whether a subject has precancerous anal lesions consisting of high-degree squamous intraepithelial lesions (HSIL) based on the calculated score value.

In a preferred embodiment of the fourth aspect of the invention, step b) calculates the score value and thus the risk of a subject being at risk of having precancerous anal lesions consisting of high-degree squamous intraepithelial lesions (HSIL) if:
- the intracellular concentration of succinyl-CoA obtained in a) is greater than about 50 ng succinyl-CoA /mg of total protein present in the biological sample; and/or
- the intracellular concentration of cobalamin obtained in a) is greater than about 150 ng of cobalamin /mg of total protein present in the biological sample.

More preferably, if:
- the intracellular content of succinyl-CoA obtained in a) is greater than about 50 ng succinyl-CoA/mg of total protein present in the biological sample; and
- the intracellular content of cobalamin obtained in a) is greater than about 150 ng of cobalamin /mg of total protein present in the biological sample.

A fifth aspect of the invention refers to a data processing apparatus/device/system comprising means for carrying out the steps a) to c) of the fourth aspect of the invention. Preferably said data processing apparatus/device/system further comprises means for detecting and/or quantifying succinyl CoA and/or cobalamin.

A sixth aspect of the invention refers to a computer program [product] comprising instructions which, when the program is executed by a computer, cause the computer to carry out the steps of the of the fourth aspect of the invention.

A seventh aspect of the invention refers to a method of treating a subject having precancerous anal lesions consisting of high-degree squamous intraepithelial lesions (HSIL), wherein said subject has been classified as having or being at risk of having high-grade squamous intraepithelial lesions (HSIL) according to any of the third to sixth aspects of the invention, and wherein said method further comprises administering to the subject an anti-cancer therapy.

The following examples merely exemplified the present invention but do not limit the same.

### Examples

### Example 1

### MATERIALS AND METHODS

### Study population

We conducted a study at the anal HPV clinics of three tertiary hospitals in Madrid, Spain (Hospital Universitario Ramón Cajal, Hospital La Paz, and Hospital Universitario Fundación Jiménez Diaz) and one in Milano, Italy (Clinic of Infectious Diseases ASST Santi Paolo e Carlo. Between January 2016 and November 2018, we included people with HIV who were >18 years of age, who were not diagnosed with anal cancer prior to enrolment, and who were either evaluated for the first time for HSIL and anal cancer screening or on follow-up, including patients with a past history of HSIL. Subjects with HSIL were those in whom HSIL was histologically demonstrated in any of the biopsies taken during the HRA procedure or with a cytologic result reported as HSIL. We also used an alternative definition of HSIL in which we excluded the cytologic HSIL results that were not histologically validated. Subjects without bHSIL were those who completed the diagnostic procedures in whom bHSIL was not demonstrated after the diagnostic work-up. We divided the whole population in a discovery cohort (Hospital Universitario Ramón y Cajal and La Paz) and a validation cohort (Fundación Jiménez Diaz and ASST Santi Paolo e Carlo Clinic).

### Anal cytologic sampling

We used the Anex Brush (Rovers Medical Devices, Netherlands) to obtain two anal samples, one was rinsed in 5 mL of RNA later in sterile Falcon tubes and stored immediately at -80ºC, and the second one was rinsed in a vial containing 20 ml of PreservCyt (Hologic, Inc., Marlborough, MA, United States) fixative medium. Cytological results were examined by the specialized cytopathologists at each center and reported following the Bethesda System as unsatisfactory; negative, atypical squamous cells of undetermined significance (ASCUS); low-grade squamous intraepithelial lesion (LSIL); atypical squamous cells, cannot exclude HSIL (ASC-H); or HSIL. The anal cytology samples were tested for HPV using the Abbott RealTime High Risk (HR) HPV assay, which detects 14 high-risk HPV types (16, 18, 31, 33, 35, 39, 45, 51, 52, 56, 58, 59, 66, and 68) and reports on HPV 16 and 18 separately from the other high-risk HPV types.

### Clinical examination, including anal cytology, HRA and histology

All participants had a digital anorectal exam followed by high-resolution anoscopy (HRA). For this procedure, the participating centers followed the Consensus Recommendations of the International Guidelines for Practice Standards (IANS) in the detection of Anal Cancer Precursors. The procedure was performed by trained physicians with a minimum of 8 years of experience in HRA (range 8-12 years). We took anal biopsies from suspicious areas revealed by HRA as acetowhite lugol-negative lesions with baby-Tischler forceps. Several experienced pathologists, all from the same institution, read the anal histology slides, with consensus discussion of all difficult or equivocal cases. The histologic results were classified according to the Lower Anogenital Squamous Terminology (LAST) project recommendations. Results were reported as negative, LSIL, HSIL, or squamous cell carcinoma. When we obtained multiple biopsies, we used the most severe result as the histological diagnosis in the analysis. We offered treatment with infrared coagulation, hyfrecation or topical imiquimod coagulation to patients in whom the biopsy revealed high-degree intraepithelial lesions (HSIL). Among patients who we evaluated as having no visual abnormalities suggestive of LSIL or HSIL on HRA examination, and who had normal anal cytology were classified as normal; otherwise, the worst histologic result was considered for the group assignment.

### DNA extraction and sequencing analysis

Cryopreserved anal samples obtained in sterile Falcon were thawed and aliquoted to 1.5 eppendorf tubes. First, these tubes were centrifuged at 4ºC 13000rpm for 30 min. The cellular pellet was resuspended in 500 µl of Phosphate-Buffered Saline (PBS) and divided into two 250 µl samples. Cellular pellets from both types of samples were treated for 30 min at 37ºC with lysozyme (20 mg/mL) in lysis buffer (QIAamp Fast DNA Stool Mini Kit) until the samples were completely homogenized. Subsequently, we added 45 µl of proteinase K and incubated at 65°C for 10 min and then at 90°C for another 10 min. We transferred the sample to a tube containing 250 µl of glass beads and vortexed it at maximum speed for 1 min. After centrifugation at 13000 rpm for 5 min, we transferred the supernatant to a new tube. Nucleic acid concentration was measured using the Qubit 2.0 Fluorometer (Life Technologies, Paisley, UK).

For each sample, the V3-V4 regions of the 16S rRNA gene were amplified and the amplicon libraries were constructed following Illumina instructions (Illumina, San Diego, CA, USA) at the FISABIO Sequencing and Bioinformatics Service, Valencia, Spain.

### Extraction of proteins, mass spectrometry analysis, and metabolites quantification

Cryopreserved anal samples were thawed at 4ºC overnight (16 hours) and centrifuged the entire content in a 1.5 ml-Eppendorf for 30 min at 13000 *rpm* and 4 °C to separate the cellular pellet. After separating the supernatant, cells were dissolved on ice with 300 µl chaotropic lysis buffer (7 M urea, 2 M thiourea, 5% 3-[(3-cholamidopropyl) dimethylammonio]-1-propanesulfonate and 10 mM 1,4-dithiothreitol). After incubation on ice, cells were further disrupted by sonication using a pin Sonicator^{®} 3000 (Misonix, New Highway Farmingdale, NY, USA) for a total time of 2 min (10 W) on ice (3 cycles x 20 sec with 1.0 min ice-cooling between each cycle). The extracts were then centrifuged for 20 min at 13000 *rpm* and 4 °C and the supernatants were then carefully aspirated (to avoid disturbing the pellet), transferred to new tubes, and stored at -80°C until use for the proteomic analysis and metabolite measurements.

The proteomic analysis was performed at the Hohenheim Universtity, Stuttgart, Germany. After peptide purification, an internal standard (peptide mixture of an enolase from yeast) was spiked before mass spectrometric analyses. The peptides were measured with a Thermo qExactive HF-X LC-MS instrument with a 90 min LC gradient. Raw data were analyzed using iMetaLab (v2.1) metaproteomics pipeline. Searches were done using the human gut integrated non-redundant gene catalog (IGC), which contains ~9.9 million genes (Li, J., Jia, H., Cai, X. et a/. An integrated catalog of reference genes in the human gut microbiome. Nat Biotechnol 32, 834-841 (2014). https://doi.org/10.1038/nbt.2942). Quantification was done by Maxquant implemented in the iMetalab pipeline resulting in label-free quantification values (LFQ). LFQ data were normalized by using the LFQ values of the enolase across all measurements. In total, 9211 quality-filtered proteins were obtained from the 280 samples. MSMS coverage ranges from 0 to 18%, with a mean value of 4 and a median of 3. The number of peptides of microbial proteins per sample range from 32 to 38208, with a mean of 1295 and a median of 795.

### Quantification of succinyl-CoA and cobalamin

Intracellular concentrations of succinyl-CoA and cobalamin were determined in triplicates in the 187 out 213 samples with enough supernatant volume after being used for proteome analysis using the succinyl-CoA Synthetase colorimetric assay Kit (ab196989) and the vitamin B12 ELISA kit (LS-F13023 de LifeSpan BioSciences). Calibration curves using succinyl-CoA (ref. S1129-5MGM, Merck Life Science S.L.U., Madrid, Spain) and vitamin B12 (or cobalamin) (ref. V6629-100MG, Merck Life Science S.L.U., Madrid, Spain) were prepared prior to the assay. The concentrations were expressed as nmol/mg cell dry weight.

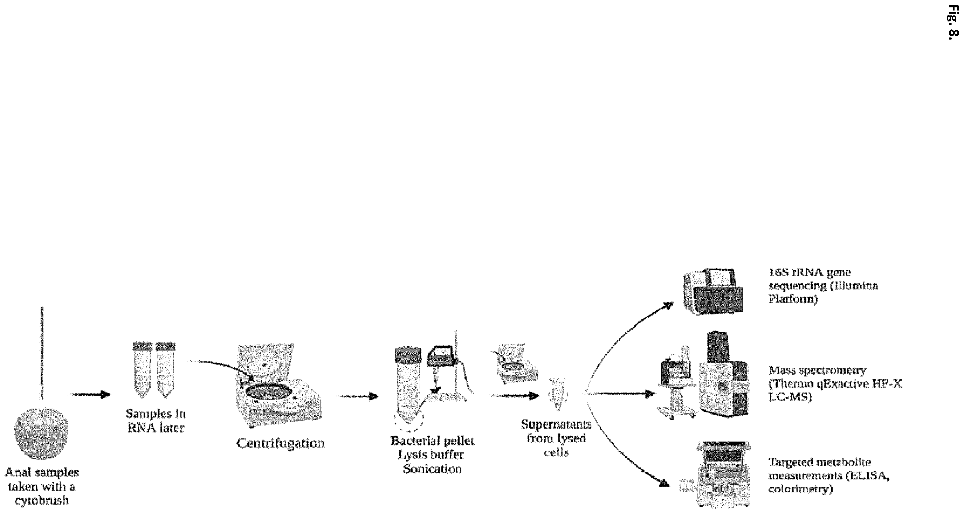

The general characteristics of the individuals providing the 187 samples did not differ from those of the parent population. The concentration of succinyl-CoA and cobalamin, measured in triplicates, was given as ng/mg protein. Prior to the assay, the protein concentration was measured using the PierceTM 660 nm Protein Assay Reagent, following the recommendation by the supplier (Thermo Scientific, Rockford, USA). **Figure** 8 summarizes the protocol.

### Bioinformatic analysis

### Taxonomic analyses based on 16S rRNA gene sequences

Sequences were analysed with QIIME 2 2020.8.0 . Raw sequence data were demultiplexed and quality filtered using the q2-demux plugin followed by denoising with DADA2 generating Amplicon Sequence Variants (ASV). Reads with low quality score, short read lengths (<50 nucleotides) were removed. All ASVs were aligned with mafft and used to construct a phylogeny with fasttree2. Potential chimeras and human sequences were also removed from the remaining sequences. Taxonomy was assigned to ASVs using the SILVA database (v.138). Samples were rarefied to 25000 sequences per sample. All statistical analyses were conducted in R (v. 4.0.2) using the microbiome (v. 1.18.0), vegan (v. 2.6-2), DESeq2 (v. 1.36.0) and phyloseq (v. 1.40.0) packages. Alpha diversity indices were calculated using the Vegan package. Beta diversity analysis was based on unweighted Unifrac distances that were determined using the vegan and Phyloseq R package and non-metric multidimensional scaling (NMDS), and sample clustering was performed. PERMANOVA test with 999 permutations was performed using the function adonis and betadisper in vegan and Phyloseq R-package to assess the differences in bacterial community structure among HSIL vs No HSIL groups. Differentially abundant ASVs were identified using the negative binomial distribution model, implemented in the DESeq2 package. P-values were calculated and adjusted by the false discovery rate (FDR). Differentially abundant ASVs presented using a volcano plot, ASVs are coloured if they pass the thresholds for FDR and Log2Fold Change and labeled to the nearest identified species/taxa.

All the sequences are publicly available in the European Nucleotide Archive database under accession number PRJEB52747.

### Proteomic signatures related to HSIL

Proteomics analysis of proteomic datasets was performed in all samples. The total number of identified proteins ranged from 2 to 3,047, being selected for further analysis of those 51 samples with >500 proteins identified, of which 12 out 51 were assigned to the HSIL group, while 39 out of 51 were assigned to the non-HSIL group. Preprocessing of the protein intensities for the 51 selected proteomes was performed as follows: a) transformation by their log₂; b) filtering of proteins, keeping those present at least on the 60% of the samples in any of the groups; c) normalization, by subtracting global median of each sample to each corresponding protein log₂₋value and d) imputation of missing values, by Probabilistic Minimum Imputation method. After preprocessing, 423 out of 8,952 identified proteins among the 51 samples were retained for further analysis. Differential abundance of proteins among the two groups were studied using the Fold Change (FC) of the mean log₂-intensities of the two groups and the p-value returned from a *moderate t-test* performed on each protein comparing both groups' log₂-intensities.

### Statistical analysis

Statistical analysis proceeded according to a prespecified statistical analysis plan. Study data were collected and managed using REDCap electronic data capture tools. We report qualitative variables as frequency distribution and quantitative variables as medians with their 25^{th}-75^{th} percentiles (P25-P75) or as means and standard deviation (SD), according to the data distribution. We performed comparisons between groups using the Chi-square test for categorical variables. Since the distribution of all the assessed variables departed from normality, we used the Mann-Whitney U test for the between-group comparisons of continuous variables.

Our primary objective was to discover and validate new biomarkers of HSIL. The biomarker selection was performed in the test cohort and was based on biological criteria (overlapping pathways identified by the proteomic analysis), methodological consistency (differences in biological pathways demonstrated by different techniques -mass spectrometry, ELISA, and colorimetry-), biological relevance (markers likely to be implicated in the oncogenic transformation) and statistical criteria (differential abundance between groups of the assessed biomarkers). We used logistic regression models to test the diagnostic accuracy of each of the selected biomarkers, alone and in combination and as continuous or categorical variables, after selection of the cut-off optimizing their sensitivity and specificity using ROC curves.

We defined our predictor "positive metabolic test" as a composite variable in which the test was positive if any of the two metabolites was detected above the selected threshold. To test the accuracy we calculated the sensitivity, specificity, positive predictive value (PPV) and NPVs, positive and negative likelihood ratios, and areas under the receiver operating characteristic curves (AUCs) for all cohorts, together with their respective 95% Cis. The AUC in the derivation (refinement) cohort was corrected for model optimism by bootstrap internal validation. To examine the model calibration, we plotted the observed vs. predicted results. Finally, to assess whether the results could be extrapolated to a different population, we calculated the HSIL probabilities estimated by a positive metabolic test in the test cohort to those observed in the external validation cohort. We pooled the data from each of the discovery and validation cohorts to calculate the net reclassification index. For the statistical analyses, we used Stata v17.0 (StataCorp LP, College Station, TX).

### Sample size

We established the sample size needed in the discovery cohort to derive a clinical decision rule that would contain a maximum of 6 predictors. To correctly quantify the predictive performance of a predictive multivariate model with respect to calibration and discrimination, at least 10 events per predictive variable are required. Hence, we estimated that we will include 60-70 cases of HSIL in the derivation cohort. As the frequency of HSIL in MSM with HIV typically ranges from 35% to 55%, we estimated that 250 individuals would be required in the discovery cohort. After we had confirmed 70 HSIL instances in the discovery cohort, so we stopped the recruitment. Because the analyses were post hoc in the validation cohort, no formal sample size calculations that are directly relevant to this analysis were undertaken a priori.

### Ethics

This study conformed to the principles of the Declaration of Helsinki and Good Clinical Practice Guidelines and was approved by the Independent Ethics Committee of the coordinating center Hospital Ramón y Cajal (ceic.hrc@salud.madrid.org, approval 461-15) and the participating centers. All the participants gave informed consent before the initiation of the study procedures.

### Results

### General characteristics

We included 213 who completed all the evaluations, the discovery cohort included 167 individuals with 70 HSIL cases, and the validation cohort included 46 individuals with 25 HSIL cases. The general characteristics of the study participants are described in Table 1. This population was representative of a middle-aged (44 years [38 - 52]) population. Most participants were MSM (94%) with HIV on ART (100%) with long-term HIV RNA suppression (48 months [24 - 91]) and an adequate immunological situation (median CD4, 728 counts/uL [496 - 930] and median CD4/CD8 ratio, 0.7 [0.5 - 0.9]). The prevalence of high-risk HPV in anal samples was 88.5%. Overall, these characteristics were comparable in the derivation and validation cohorts.

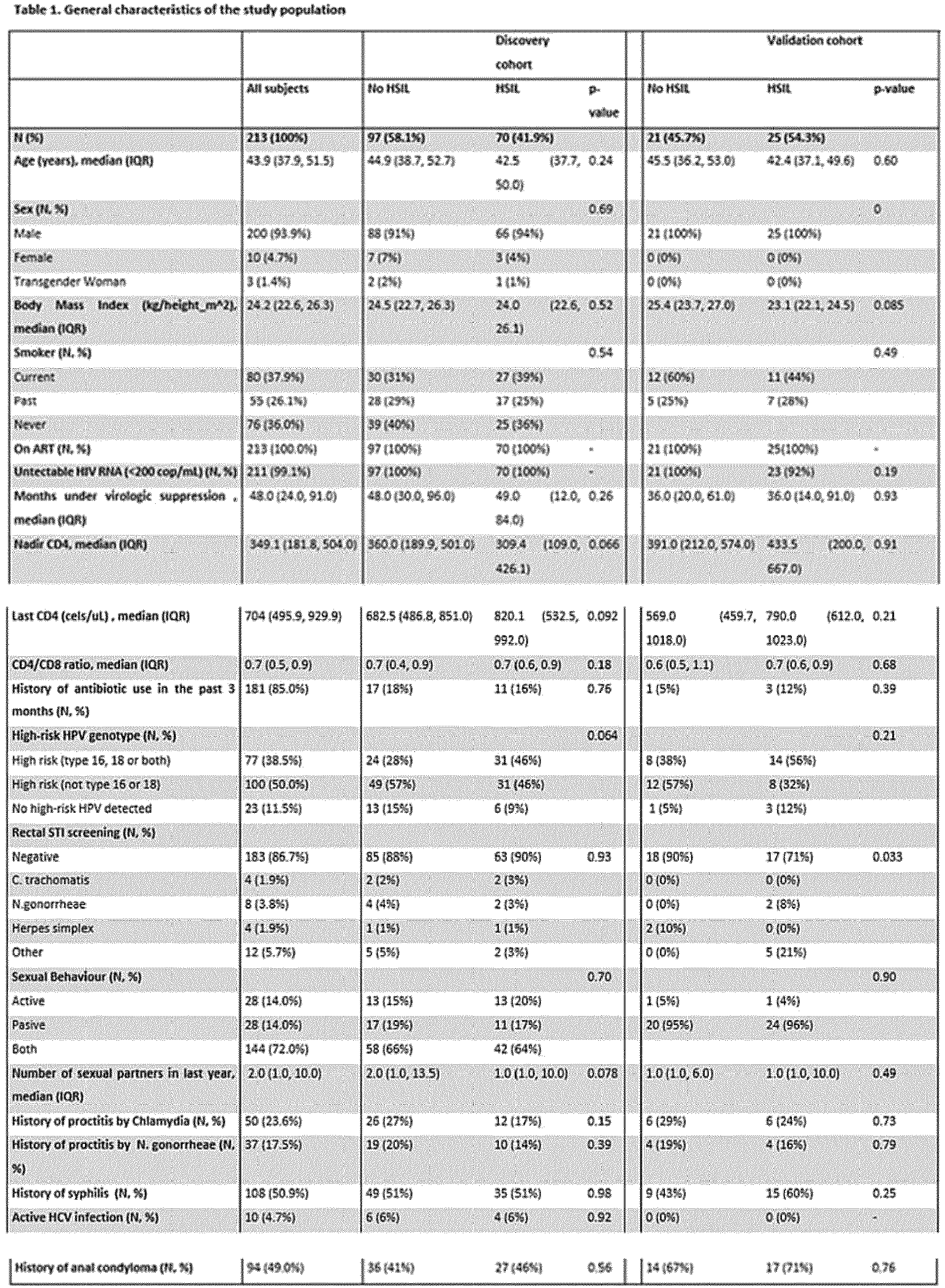

### Differences in the gut microbiota composition appeared more relevant at the functional level

We first studied the compositional level obtained by the 16S rRNA gene sequencing analysis, which allows determining the overall microbiota composition including live, dead, active, and inactive bacteria. We did not detect clear differences between groups in alpha diversity, alpha diversity, a metric of the richness and evenness of bacterial taxa within a community, or beta diversity, which informs about the extent of variation in microbial communities' composition.

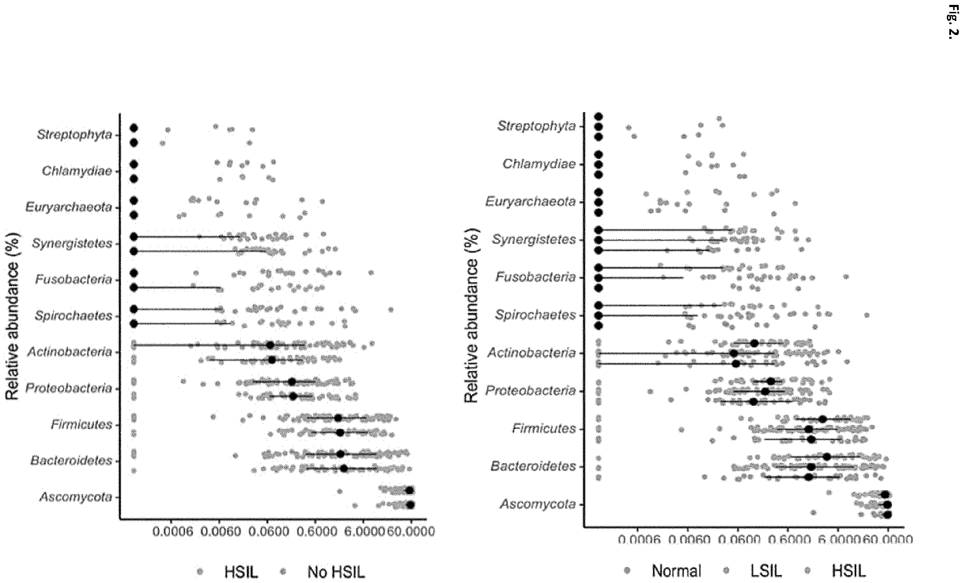

Then, we determined the signatures associated with HSIL on the most abundant taxonomic groups, i.e., those with an abundance >10% in at least one sample, without differences between study groups (Figure 2A). Then, when we plotted the ASVs fold changes between groups, subjects with HSIL were enriched in Prevotella copri, while showing depletion of Streptococcus periodonticum, Dialister succinatiphilus, Prevotella stercorea, Sneathia sanguinegens, Fusobacterium goniadiaformans and Anaerovibrio lipolyticus (Figure 2B). We had previously appreciated in a different cohort that Sneathia sanguinegens is significantly depleted in individuals with HSIL. However, when we compared the abundance of these bacterial species in each group (figure 6) we found that most differences were driven by a limited number of observations, challenging the use of these taxa as potential biomarkers of HSIL.

### Functionally active bacteria annotated from the proteomic profiles

In total, 9211 quality-filtered proteins were obtained and associated with 13 distinct phyla and up to 135 species. The relative-abundance of the annotated peptides at the lower common ancestor (LCA) was used to calculate the compositional dissimilarity between the samples using the Bray-Curtis dissimilarity and the variance associated between the different categories was evaluated using the PERMANOVA test. No significant differences were observed between patients with HSIL compared to those without HSIL and HSIL vs. LSIL. Vs. no dysplasia (Figure 1). Additionally, no clear discrimination was observed between the different evaluated categories in the ordination analysis. Overall, 79 % of annotated peptides belonged to Eukaryotes while 20% and 0.02% belonged to Bacteria and Archaea respectively. At the phylum level, Ascomycota (49%) was the most abundant group, followed by Bacteroidetes 8.3%, Firmicutes (4.9%) and Proteobacteria (0.5%). Additionally, unclassified phyla represented 35% of the peptides. No significant differences were found across all taxonomic levels in subjects with HSIL vs those without HSIL and HSIL vs. LSIL. Vs. no dysplasia (Kruskal-wallis test p>0.05, p adjusted by BH). However, subjects with HSIL had a higher abundance of Bacteroidetes, Firmicutes and Actinobacteria members compared to those subjects with LSIL and no dysplasia (Figure 2).

### Proteomic signatures related to HSIL

Then, we analyzed the functional profiles of the anal microbiota based on the expressed proteins **(****Figure 3A****).** Overall, the most represented functions related to translation, ribosomal structure and biogenesis, carbohydrate transport and metabolism, and energy production and metabolism. Then, when comparing the proteomic profiles in patients with HSIL vs. those without HSIL, we appreciated marked differences. Six proteins were overexpressed in the HSIL group **(****Figure 3B-E****),** including (i) a glyceraldehyde-3-phosphate dehydrogenase (GapN), which over-expression has been associated with several tumors, including HPV-related cervical cancer (ii) a phosphoglycerate kinase 1 (PGK1), an ATPgenerating enzyme in the glycolytic pathway, which over-expression represents a relevant molecular mechanism of HPV-related oncogenesis iii) a NADH ubiquinone oxidoreductase which over-expression has been associated to colorectal cancer onset< (iv) an enolase (ENO), which over-expression has been associated to many cancers and is also used by pathogenic bacteria to invade the extracellular matrix (v) a ribosomal Protein L2 which over-expression has been associated to human cancers; and (vi) biopolymer transport protein ExbD, associated to biopolymer, iron, and vitamin B12 uptake. The 6 overrepresented proteins were also visualized by Principal Components Analysis (PCA), showing a distinct cluster based on the expression of these six proteins in the HSIL vs. non-HSIL groups **(****Figure 3C****).**

**Table 2. Proteins found to be overexpressed in HSIL compared to non-HSIL groups.**

| **Protein ID** | **Fold change/SD (HSIL vs non-HSIL)** | **COG name** | **Links with cancer pathogenesis** |
|---|---|---|---|
| T2D-8A_GL0079056 | 3.62 | Biopolymer transport protein, ExbD | Identified as a potential therapeutic target for gastric cancer ^{25,26}. |
| O2.UC49-0_GL0126921 | 3.16 | NADH:ubiquinone oxidoreductase 27 kD subunit (chain C), GapN | Implicated in H2O2 production and in the apoptosis, autophagy, and survival mechanisms of several cancers ^{20,21}. |
| MH0184_GL008900 9 | 3.26 | Glyceraldehyde-3-phosphate dehydrogenase, GapN | cGMP/PKG pathway linked to breast cancer stemness and metastasis ²⁷ , cervical cancer ¹⁹ and activated by HPV16 ²⁸. |
| V1.CD6-0-PT_GL0019483 | 3.21 | NA | - |
| MH0012_GL018449 4 | 4.34 | 3-Phosphoglycerate kinase, PKG1 | cGMP/PKG pathway linked to breast cancer stemness and metastasis ²⁷ , cervical cancer ¹⁹ and activated by HPV16 ²⁸. |
| MH0041_GL007186 6 | 4.34 | Enolase, ENO | Regulates stem-cell properties in many cancers ^{22,29} and is a biomarker of non-small cell lung cancer ³⁰. |

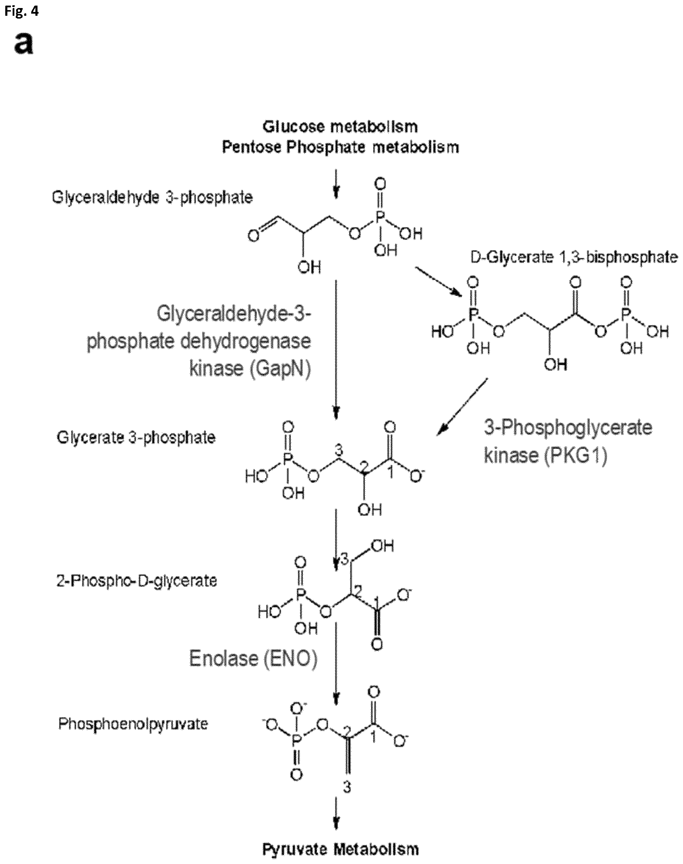

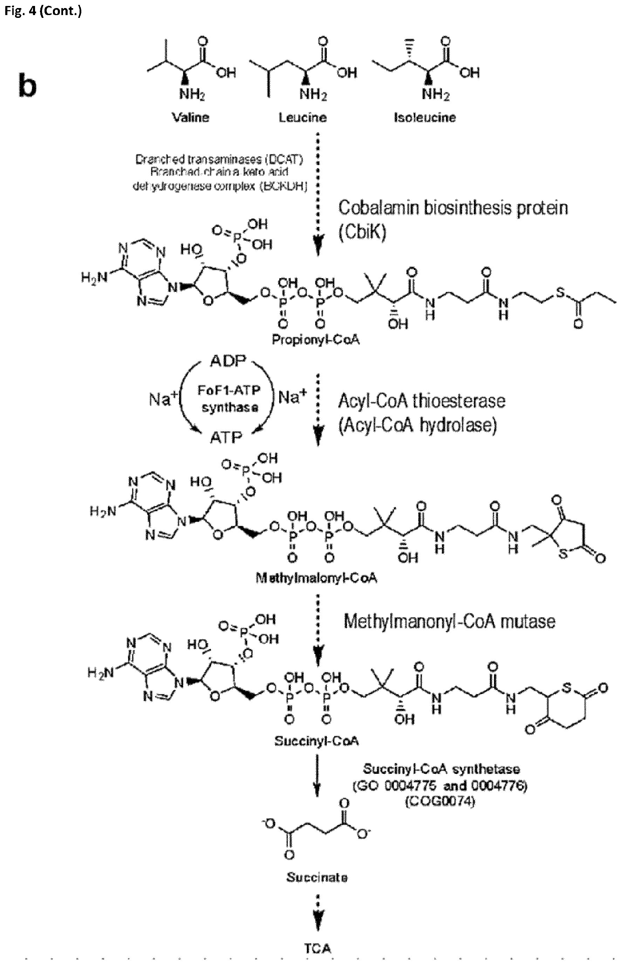

Importantly, three of the protein biomarkers, namely GapN, PGK1, and ENO are part of the glycolysis pathway **(****Figure 4****),** The three proteins contribute to the transformation of glyceraldehyde 3-phosphate, an intermediate in both glycolysis and gluconeogenesis, which is a main product of the pentose phosphate and glucose metabolism, into phosphoenolpyruvate, which is a main product of the pyruvate metabolism. The glyceraldehyde 3-phosphate is partially produced from the pentose phosphate pathway (PPP), which comprises two phases: the non-oxidative phase, which produces reduction equivalents, and the oxidative phase, which produces 5-carbon sugars for the synthesis of nucleotides. As key enzymes in the oxidative phase we checked the proteome data for expression of the glucose-6-phosphate dehydrogenase (G6PD; EC 1.1.1.49), and phosphogluconate dehydrogenase (PGD), and in the non-oxidative phase, we examined the expression of transketolase (TKT; EC 2.2.1.1). We found G6PD and PGD not to be expressed or identified in the proteomes of subjects with HSIL or LSIL. In contrast, expression levels of transketolase (protein ID MH0119_GL0116660) were increased in the HSIL group compared to the non-HSIL group, although the differences did not reach the threshold of statistical significance (p=0.37).

Then, we reasoned that segregating the individuals by their degree of dysplasia (LSIL or HSIL) will help to gain more resolution on the relevant pathways. Hence, we performed additional analyses to explore other categorizations considering three groups-no dysplasia, LSIL, and HSIL-and including in the HSIL group the cytologic HSIL not biopsy-proven. Compared to subjects with LSIL and no dysplasia, ribosomal protein L7/L12, was overexpressed in subjects with bHSIL (p-value < 0.033). Additionally, 6 proteins, including OmpC outer membrane porins, CbiK cobalamin biosynthesis proteins, FoF1-type ATP synthases, acyl-CoA hydrolases, methylmalonyl-CoA mutases, and hypothetical proteins, previously linked to cancer pathogenesis were overexpressed in **HSIL** compared to LSIL and no dysplasia groups **(Table 3; p-value <0.0386).** For example, the 50S ribosomal protein L7/L12 (rpIL) has been found to be an effective adjuvant for enhancing the therapeutic response to dendritic cell (DC)-based tumor immunotherapy.

**Table 3. Proteins overexpressed in HSIL compared to LSIL and normal groups.**

| | **Fold change/SD** | | | | | |
|---|---|---|---|---|---|---|
| **Protein ID** | **bAINc at (HSIL vs Norm al)** | **bAINc at (HSIL vs LSIL)** | **cAINc at (HSIL vs Norm al)** | **cAINcat (HSIL** vs **LSIL)** | **COG name** | **Links with cancer pathogenesis** |
| 553171.HMPREF0648 _0146 | 2,582 | 3,3924 | 0 | 0 | Ribosomal protein L7/L12 (COG0222) | Enhancement of the therapeutic response to dendritic cell (DC)-based tumor immunotherapy ³². |
| V1.UC58-0_GL0027568 | 0 | 0 | -2,781 | -2,7111 | Outer membrane porin, OmpC (COG3203) | Assigned to bacteria from the Firmicutes phylum, namely, from Peptoniphilaceae, Fubacteriaceae, Ruminococcaceae, and to Bacteroidota, namely, Prevotellaceae family. The overexpression of OmpC proteins in the large intestinal microbiota have been shown to be related to sporadic colorectal cancer ³³. OmpC indirectly related to the cobalamin transport ^{34,35}. |
| NLM027_GL0041017 | 0 | 0 | 2,7688 | 2,9348 | Cobalamin biosynthesis protein CbiK, Co²⁺ chelatase (COG4822) | Cobalamin is necessary for the conversion of branched amino acids linked to cancer pathogenesis ³⁶. |
| MH0357_GL0122175 | 0 | 0 | 2,9312 | 2,742 | FoF1-type ATP synthase, beta subunit (COG0055) | Involved in the pathogenesis of liver metastasis and colorectal cancer ^{37,38}. |
| MH0006_GL0032163 | 0 | 0 | 2,8426 | 2,7532 | Acyl-CoA hydrolase (COG0427) | Thyosterase that regulates cellular levels and proper ratios of free and activated fatty acids and CoA-SH. Abnormal metabolism of lipids and fatty acids is observed during gastric carcinoma progression ^{39,40}. Overexpressed in gastric cancer, significantly correlated with poor prognosis in gastric cancer 41. |
| MH0006_GL0032162 | 0 | 0 | 2,6046 | 2,7924 | Methylmalonyl-CoA mutase, N-terminal domain/subunit (COG1884) | Related to the catabolism of propionyl-CoA, branched-chain amino acids, and the β-oxidation of odd-chain fatty acids ⁴², linked to cancer pathogenesis ⁴³. |
| MH0053_GL0055730 | 0 | 0 | 3,3153 | 3,2741 | Hypothetical | - |

The taxonomic analysis of these proteins is summarized in **Table 4.** The proteins overexpressed in HSIL are assigned to bacteria from the Firmicutes phylum, namely, from Peptoniphilaceae, Eubacteriaceae, Ruminococcaceae families, and to Bacteroidota, namely, Prevotellaceae family. We also found being underexpressed in HSIL an outer membrane porin OmpC (V1.UC58-0_GL0027568). These proteins are assigned to bacteria from the Firmicutes phylum, namely, from Peptoniphilaceae, Eubacteriaceae, Ruminococcaceae, and to Bacteroidota, namely, Prevotellaceae family. The overexpression of OmpC proteins in the gut microbiota has been linked to sporadic colorectal cancer. OmpC has been also demonstrated to be indirectly related to cobalamin transport.

**Table 4. Taxonomy of proteins overexpressed in HSIL within the individuals with biopsy and cytology as diagnostic test.**

| **Protein ID** | **Taxonomy** |
|---|---|
| 553171.HMPREF0648_0146 | Firmicutes: Peptoniphilaceae, Eubacteriaceae, Ruminococcaceae |
| | Bacteroidota: Prevotellaceae |
| V1.UC58-0_GL0027568 | Firmicutes: Selenomonadaceae, Acidaminococcaceae |
| | Bacteroidota: Rikenellaceae, Prevotellaceae |
| NLM027_GL0041017 | Bacteroidota: Bacteroidaceae, Prevotellaceae |
| MH0357_GL0122175 | Firmicutes: Veillonellaceae, Ruminococcaceae |
| MH0006_GL0032163 | Firmicutes: Lachnospiraceae |
| | Bacteroidota: Prevotellaceae |
| MH0006_GL0032162 | Firmicutes: Veillonellaceae |
| | Bacteroidetes: Prevotellaceae |
| MH0053_GL0055730 | Firmicutes: Ruminococcaceae |
| | Bacteroidota: Prevotellaceae |
| | Actinobacteria: Bifidobacteriaceae |

Overall, the proteomic analysis of samples categorized by the degrees of dysplasia, revealed that the development of HSIL is directly related to overexpression of a subset of proteins related to the metabolism of acyl-CoA, branched-chain amino acids (e.g. valine, isoleucine), cobalamin biosynthesis and transport, and ATP synthesis that converged in the production of methylmalonic acid, that is a precursor for succinyl-CoA and gluconeogenesis **(Figure 10).** Alterations in all these proteins have been related to tumor progression **(Table 3).** The taxonomic analysis **(Table 4)** suggests that the bacteria most likely expressing such proteins belong to the phyla Firmicutes (Peptoniphilaceae, Eubacteriaceae, Veillonellaceae, Ruminococcaceae, Lachnospiraceae, Veillonellaceae, and Ruminococcaceae families), Bacteroidota (Bacteroidaceae, Prevotellaceae families), and Actinobacteria (Bifidobacteriaceae families).

### Metabolomic analysis of the key metabolites linked to bHSIL

The proteomic analysis found over-expression of cobalamin, acyl-CoA, and methylmalonyl-CoA, which together contribute to the succinyl-CoA pool. To verify this, we quantified the intracellular concentrations of succinyl-CoA in the anal microbiota. Subjects with HSIL exhibited higher levels of succinyl-CoA and cobalamin than those without HSIL.

| **Protein ID** | **Fold change/S D (HSIL vs non-HSIL)** | **COG name** | **Links with cancer pathogenesis** | **Taxonomy** |
|---|---|---|---|---|
| T2D-8A_GL007905 6 | 3.62 ↑ | Biopolymer transport protein, ExbD | Identified as a potential therapeutic target for gastric cancer ^{28,29}. | *Spirochaetes: Brachyspiraceae* |
| | | | | *Firmicutes: Eubacteriaceae* |
| | | | | *Bacteroidetes: Prevotellaceae* |
| O2.UC49-0_GL0126921 | 3.16 ↑ | NADH:ubiquinone oxidoreductase 27 kD subunit (chain C), GapN | Implicated in H2O2 production and in the apoptosis, autophagy, and survival mechanisms of several cancers ^{23,24}. | *Firmicutes: Clostridiales* Family |
| | | | | XII, *Eubacteriaceae* |
| | | | | *Bacteroidetes: Prevotellaceae,* |
| | | | | *Porphyromonadaceae,* |
| | | | | *Flavobacteriaceae* |
| MH0184_GL0 089009 | 3.26 ↑ | Glyceraldehyde-3-phosphate dehydrogenase, GapN | cGMP/PKG pathway linked to breast cancer stemness and metastasis ³⁰ , cervical cancer ²² and activated by HPV16 ³¹. | *Bacteroidetes: Prevotellaceae* |
| | | | | *Euryarchaeota:* |
| | | | | *Methanobacteriaceae* |
| | | | | *Firmicutes:* unclassified |
| | | | | *Proteobacteria:* unclassified |
| V1.CD6-0-PT_GL001948 3 | 3.21 ↑ | NA | - | *Bacteroidetes: Prevotellaceae,* |
| | | | | *Bacteroidaceae* |
| | | | | *Proteobacteria:* |
| | | | | *Succinivibrionaceae* |
| | | | | *Firmicutes: Erysipelotrichaceae,* |
| | | | | *Selenomonadaceae* |
| MH0012_GL0 184494 | 4.34 ↑ | 3-Phosphoglycerate kinase, PKG1 | cGMP/PKG pathway linked to breast cancer stemness and metastasis ³⁰ , cervical cancer ²² and activated by HPV16 ³¹. | *Bacteroidetes: Prevotellaceae* |
| | | | | *Firmicutes: Ruminococcaceae,* |
| | | | | *Clostridiales* |
| MH0041_GL0 071866 | 4.33 ↑ | Enolase, ENO | Regulates stem-cell properties in many cancers ^{25,32} and is a biomarker of non-small cell lung cancer ³³. | *Bacteroidetes: Prevotellaceae* |
| | | | | *Firrmicutes: Lactobacillaceae,* |
| | | | | *Lachnospiraceae* |
| | | | | *Spirochaetes: Brachyspiraceae* |
| | | | | *Actinobacteria:* |
| | | | | *Bifidobacteriaceae* |
| 553171.HMPR EF0648_0146 | 3.39 ↑ | Ribosomal protein L7/L12 (COG0222) | Enhancement of the therapeutic response to dendritic cell (DC)-based tumor immunotherapy ³⁴. | *Firmicutes: Peptoniphilaceae, Eubacteriaceae,* |
| | | | | *Ruminococcaceae* |
| | | | | *Bacteroidota: Prevotellaceae* |
| V1.UC58-0_GL0027568 | -2.78 ↓ | Outer membrane porin, OmpC (COG3203) | Assigned to bacteria from the Firmicutes phylum, namely, from Peptoniphilaceae, Eubacteriaceae, Ruminococcaceae, and to Bacteroidota, namely, Prevotellaceae family. OmpC is indirectly related to the cobalamin transport ^{35,36}. | *Firmicutes: Selenomonadaceae, Acidaminococcaceae* |
| | | | | *Bacteroidota: Rikenellaceae, Prevotellaceae* |
| NLM027_GL0 041017 | 2.76 ↑ | Cobalamin biosynthesis protein CbiK, Co²⁺ chelatase (COG4822) | Cobalamin is necessary for the conversion of branched amino acids linked to cancer pathogenesis ³⁷. | *Bacteroidota: Bacteroidaceae, Prevotellaceae* |
| MH0357_GL0 122175 | 2.93 ↑ | FoF1-type ATP synthase, beta subunit (COG0055) | Involved in the pathogenesis of liver metastasis and colorectal cancer ^{38,39}. | *Firmicutes: Veillonellaceae, Ruminococcaceae* |
| MH0006_GL0 032163 | 2.84 ↑ | Acyl-CoA hydrolase (COG0427) | Thyosterase that regulates cellular levels and proper ratios of free and activated fatty acids and CoA-SH. Abnormal metabolism of lipids and fatty acids is observed during gastric carcinoma progression ^{40,41}. Overexpressed in gastric cancer, significantly correlated with poor prognosis in gastric cancer ⁴². | *Firmicutes: Lachnospiraceae* |
| | | | | *Bacteroidota: Prevotellaceae* |
| MH0006_GL0 032162 | 2.60 ↑ | Methylmalonyl-CoA mutase, N-terminal | Related to the catabolism of propionyl-CoA, branched-chain amino acids, and the β-oxidation | *Firmicutes: Veillonellaceae* |
| | | | | *Bacteroidetes: Prevotellaceae* |
| | | domain/subunit (COG1884) | of odd-chain fatty acids ⁴³, linked to cancer pathogenesis ⁴⁴. | |
| MH0053_GL0 055730 | 3,31 ↑ | Hypothetical | - | *Firmicutes: Ruminococcaceae* |
| | | | | *Bacteroidota: Prevotellaceae* |
| | | | | *Actinobacteria: Bifidobacteriaceae* |

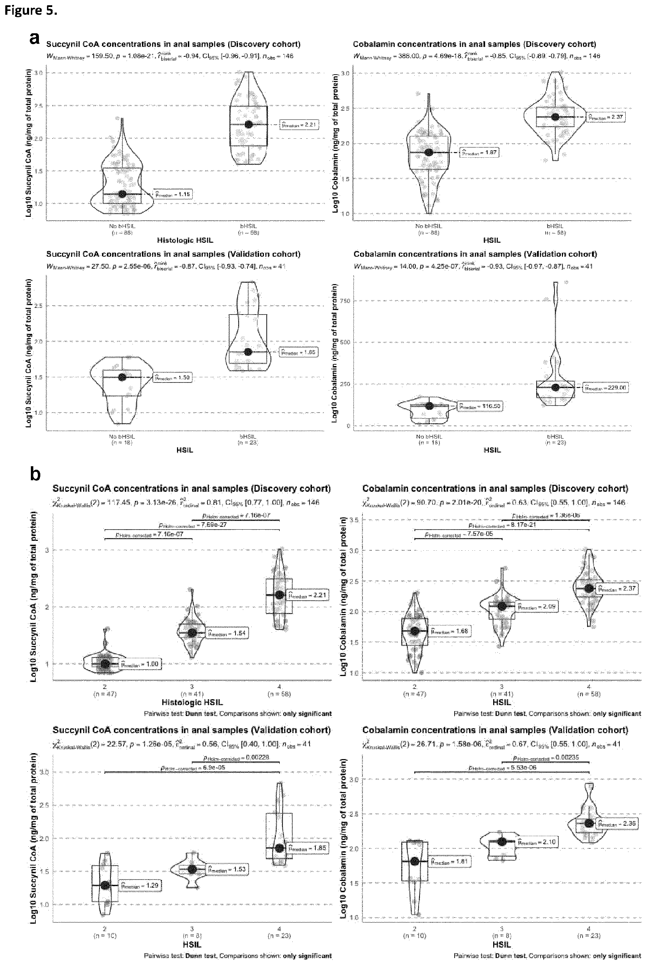

**Figure 5****).** In addition, we appreciated a gradient according to the degree of lesion: the median succinyl-CoA and cobalamin levels were 16.2 and 4.9 times higher than those in subjects with normal results, and 4.6 and 1.9 times higher compared to those with LSIL.

### Diagnostic accuracy of the anal cytology compared to the metabolic parameters

Then, we calculated the diagnostic accuracy to diagnose bHSIL of the reference technique, the anal cytology and compared it with that of succinyl coA and cobalamin in the test cohort. As expected, abnormal cytology was highly sensitive (sensitivity = 91.2%) but poorly specific (specificity = 34.1%) to detect individuals with bHSIL, failing to correctly classify 40% of participants. In contrast, when we combined succinyl coA and cobalamine as continuous variables, the sensitivity, specificity, AUC, and accuracy, were excellent, with an AUC of 0.971 and correct discrimination in 90% of participants **(Table 5** and **Figure 6a****).**

**Table 5. Diagnostic values for each diagnostic approach to predict bHSIL**

| | **Abnormal cytology** | **SucCoA Cobalamine continuous)^{∗}** | **and A: SucCoA >50 (log2 ug/mL** | **B: Cobalamin >150 ug/mL** | **A/B** + **A&B -** | **vs. A/B** + **vs. A&B** - |
|---|---|---|---|---|---|---|
| ***Cohort*** | **Derivation** | **Derivation** | **Derivation** | **Derivation** | **Derivatio n** | **Validatio n** |
| **Sensitivity** | 91.2% | 87.9% | 87.9% | 79.3% | 96.6% | 95.7% |
| **Specificity** | 34.1% | 92.1% | 87.5% | 92.0% | 81.8% | 83.3% |
| **Roc area** | 0.627 | 0.971 | 0.877 | 0.857 | 0.892 | 0.895 |
| **Positive predictive value** | 48.1% | 87.9% | 82.3% | 86.8% | 77.8% | 88.0% |
| **Negative predictive value** | 85.3% | 92.1% | 91.7% | 87.1% | 97.3% | 93.8% |
| **Accuracy** (% **correctly classified)** | 59.9% | 90.1% | 87.7% | 87.0% | 87.7% | 90.2% |

Then, we used the AUCs to select the cut-offs with optimal sensitivity and specificity. When we considered a metabolic test as a composite variable combining succinyl CoA and cobalamin levels above their cutoffs (succinyl-coA >50 ng/mg or cobalamin >150 ng/mg) the positivity of one and both markers predicted a nearly 40-fold increased risk of underlying bHSIL (OR 38.6, 95% Cl 7.9 - 187.7) and 740-fold increased risk (OR 738, 95% Cl 100.2 - 137.4) higher risk of bHSIL, respectively **(Table 5).** Importantly, this effect was also independent of the anal cytology test, even after adjustment for other variables previously reported to predict bHSIL, including age, tobacco use, current CD4 counts, anal infection by high-risk HPV genotypes or proctitis by *Chlamydia trachomatis* **(Table 6).** Sensitivity analyses excluding subjects with cytologic HSIL but not histologically confirmed HSIL yielded similar results. Because a semi-quantitative test might more easily become more readily implemented as a diagnostic test, we selected the composite variable, defined as succinyl CoA >50 ug/mL or cobalamin >150 ug/mL for validation purposes. This approach showed a sensitivity of 96.6%, a specificity of 81.8%, a positive predictive value of 77.8%, and a negative predictive value of 97.3% **(Table 5).** Last, we validated this diagnostic model in the 46 individuals in the external validation cohort and found similar diagnostic accuracy than in the discovery cohort.

**Table 6. Logistic regression models of diagnostic markers predicting bHSIL**

| **Variables predictive of bHSIL** | **Odds ratio** | **[95% conf. interval]** | **P value** |
|---|---|---|---|
| *Univariable* | | | |
| Abnormal anal cytology | 5.4 | 1.9, 14.6 | 0.001 |
| Succinyl-CoA (log2 ng/mg) | 11.1 | 4.8,25.4 | <0.0001 |
| Cobalamin (log2 ng/mg) | 15.0 | 6.4,34.8 | <0.0001 |
| *Composite Succinyl-CoA* + *Cobalamin, univariable* | | | |
| One positive vs. negative | 38.6 | 7.9,187.7 | <0.0001 |
| Both positive vs. negative | 738 | 100.2, 437.4 | <0.0001 |
| *Multivariable* | | | |
| Succinyl-CoA >50 ng/mg vs ≥50 ng/mg | 30.4 | 8.8, 104.8 | <0.0001 |
| Cobalamin >150 ng/mg vs ≥150 ng/mg | 25.4 | 7.1, 90.8 | <0.0001 |
| *Adjusted effect for composite Succinyl-CoA* + *Cobalamin^{∗}* | 126 | 27.8, 570.9 | <0.0001 |
| Positive vs. Negative | 424.7 | 58.3, 3094.8 | <0.0001 |

| | | | |
|---|---|---|---|
| ^{∗}Adjusted for: presence of an abnormal anal cytology, age, tobacco use, current CD4 counts, presence of high-risk HPV genotypes, history of *Chlamydia trachomatis* proctitis. | | | |

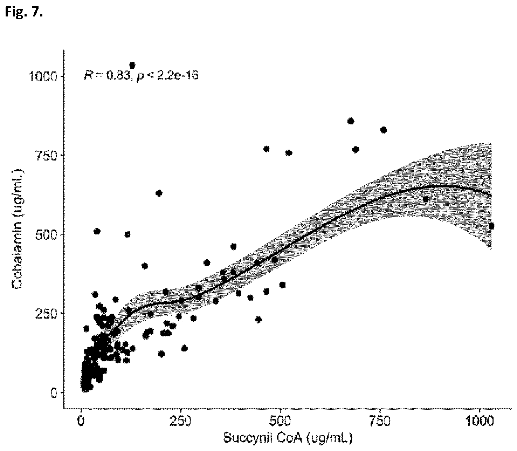

Finally, the calibration analysis yielded a global accuracy of the bacterial markers adjusted for optimism after bootstrap validation of AUC 0.896 (0.853-0.944) (Fig. 6b). The models showed an optimal calibration, as indicated by the fact that the observed vs. predicted probabilities were very close in the calibration plots. Succinyl CoA and cobalamin strongly correlated (Rho 0.83, p <0.0001), but independently contributed to the prediction of bHSIL **(Table 6** and **Figure 7****).**

As explained before, a major concern with the current screening approach based on anal cytologies is the high rate of false-positive results. In the derivation cohort, from 98 individuals in whom HSIL was ruled out, 60 (61.2%) had abnormal cytology and then were false positives who would have been referred to HRA in routine practice. The metabolic test reclassified from false positive to true negative results 49 (81.9%) of these 98 false-positive cytologic results. In return, 6 cases were incorrectly classified as bHSIL by the metabolic test despite normal cytology. **Figure 6c** illustrates the net reclassification indexes.

### Example 2. Protocol 1 for the analysis of samples of Dacron-tipped swab.

### Solutions

Chaotropic lysis buffer: 7 M urea, 2 M tiourea, 5% 3-[(3-cholamidopropyl) dimethylammonio]-1-propanesulfonate (CHAPS) and 10 mM 1,4-dithiothreitol.

The following lysis buffer, prepared immediately prior to used, can be also used. It consists in combining 2 volume reactive 1 (20% SDS / H2O)^{∗}, 1 volume reactive 2 (40 mM TCEP / 400 mM TEAB) ^{∗} and 1 volume reactive 3 (80 mM CAA / 400 mM TEAB) ^{∗}. Abbreviations as follows: SDS, Sodium dodecyl sulfate; TCEP, Tris(2-carboxyethyl)phosphine; TEAB, Triethylammonium bicarbonate; CAA, Chloroacetamide.

### Protein extraction method

1. Dacron-tipped swab is inserted into the anal canal.
2. Once removed, it is inserted in a 1.5 mL-Eppendorf with 300 µl chaotropic lysis buffer.
3. The Dacron-tipped swab moves in rotation for 1 minute.
4. Incubate the sample (the 1.5 mL-Eppendorf containing 300 µl chaotropic lysis and the dacron-tipped swab) at 60ºC for 30 minutes.
5. Allow to cool for 1 minute on ice.
6. Centrifuge for 20 min at 13000 rpm at room temperature and transfer carefully the supernatant (to avoid disturbing the pellet) to a new 1.5 mL-Eppendorf.
7. The supernatant can be stored at 4ºC or -20ºC until analysis.
8. Prior to the analysis of the metabolites (succinyl-CoA or cobalamine) by any analytical platform (ELISA platform, colorimetric/fluorimetric or mass spectrometry analysis), the concentration of protein is measured. For that, the PierceTM 660 nm protein Assay Reagent (Thermo SCIENTIFIC, ref. 22660) is recommended.
9. Measure the concentration of succinyl-CoA or cobalamine and refer it to the concentration of proteins. Succinyl CoA and cobalamin levels above their cutoffs (>50 ng succinyl-CoA /mg protein or >150 ng cobalamin /mg protein) are associated to increase risk.

### Example 3 Protocol 2 for the analysis of samples of Dacron-tipped swab.

The method above can be adapted to different lysis buffers and instead of chemical-thermal lysis a chemical-sonication lysis can be used.

### Solutions

Chaotropic lysis buffer: 7 M urea, 2 M tiourea, 5% 3-[(3-cholamidopropyl) dimethylammonio]-1-propanesulfonate (CHAPS) and 10 mM 1,4-dithiothreitol.

The following lysis buffer, prepared immediately prior to used, can be also used. It consists in combining 2 volume reactive 1 (20% SDS / H2O)^{∗}, 1 volume reactive 2 (40 mM TCEP / 400 mM TEAB) ^{∗} and 1 volume reactive 3 (80 mM CAA / 400 mM TEAB) ^{∗}. Abbreviations as follows: SDS, Sodium dodecyl sulfate; TCEP, Tris(2-carboxyethyl)phosphine; TEAB, Triethylammonium bicarbonate; CAA, Chloroacetamide.

### Protein extraction method

1. Dacron-tipped swab is inserted into the anal canal.
2. Once removed, it is inserted in a 1.5 mL-Eppendorf with 300 µl chaotropic lysis buffer.
3. The Dacron-tipped swab moves in rotation for 1 minute, after which the dacron-tipped swab is removed.
4. Sonicate the sample using a pin sonicator (for example, Sonicator^{®} 3000, Misonix, New Highway Farmingdale, NY, USA) for a total time of 2 min (10 W) on ice (3 cycles x 20 sec with 1.0 min ice-cooling between each cycle).
5. Allow to cool for 1 minute on ice.
6. Centrifuge for 20 min at 13000 rpm at room temperature and transfer carefully the supernatant (to avoid disturbing the pellet) to a new 1.5 mL-Eppendorf.
7. The supernatant can be stored at 4ºC or -20ºC until analysis.
8. Prior to the analysis of the metabolites (succinyl-CoA or cobalamine) by any analytical platform (ELISA platform, colorimetric/fluorimetric or mass spectrometry analysis), the concentration of protein is measured. For that, the PierceTM 660 nm protein Assay Reagent (Thermo SCIENTIFIC, ref. 22660) is recommended.
9. Measure the concentration of succinyl-CoA or cobalamine and refer it to the concentration of proteins. Succinyl CoA and cobalamin levels above their cutoffs (>50 ng succinyl-CoA /mg protein or >150 ng cobalamin /mg protein) are associated to increase risk.

## Claims

1. An *in vitro or ex vivo* use of the intracellular concentration of succinyl CoA and cobalamin obtained from the microbial cells of a biological sample comprising anal microbial cells, obtained or isolated from the anal canal of a subject in need thereof, preferably a human subject, as microbiota-associated markers of high-grade squamous intraepithelial lesions (HSIL).

2. The in vitro use of claim 1, wherein the biological sample is an anorectal epithelium sample, preferably an anorectal epithelium sample taken from a human subject.

3. The in vitro use according to claim 2, wherein the anorectal epithelium sample is collected or isolated from the subject by using any of the list consisting of cytobrushes with and without spatulas, cytopicks, cotton, Dacron, preferably a dacron-tipped swab, rayon, or a swab such as a nylon-flocked (NF)-swab.

4. The in vitro use of any of claims 1 or 2to 3, wherein the biological sample is an anorectal epithelium sample taken from a human subject, and wherein the subject is a human male, more preferably a human male with HIV (PWH), also preferably a human male that has sex with men, even more preferably a human male with HIV who has sex with men.

5. A method for quantifying the intracellular concentration of succinyl CoA and/or cobalamin present in a biological sample, preferably a human biological sample, comprising anal microbial cells, obtained or isolated from the anal canal of a subject in need thereof, the method comprising:
a. Lysing the microbial cells of the biological sample to obtain the intracellular content of the cells; and
b. Quantifying the intracellular concentration of succinyl CoA and/or cobalamin from the intracellular content of the cells.

6. The method according to any claim 5, wherein the quantification of step b) is carried out by a method selected from the list consisting of ELISA, colorimetric/fluorimetric, nephelometry or mass spectrometry analysis.

7. The method according to any of claims 5 to 6, wherein the biological sample is a human anorectal epithelium sample preferably collected by a swab.

8. The method according to any of claims 5 to 7, wherein the biological sample is an anorectal epithelium sample taken from a human subject, and wherein the subject is a human male, more preferably a human male with HIV (PWH), also preferably a human male that has sex with men, even more preferably a human male with HIV who has sex with men.

9. A method for determining a risk of a subject having precancerous anal lesions consisting of high-degree squamous intraepithelial lesions (HSIL), the method comprising:
a. quantifying the intracellular concentration of the microbiota-associated markers succinyl CoA and/or cobalamin present in a biological sample in accordance with any of claims 5 to 8; and
b. classifying the subject as presenting an increased or reduced risk of having precancerous anal lesions consisting of high-degree squamous intraepithelial lesions (HSIL) based on the concentration obtained in a) for each of the microbiota-associated markers or its combination.

10. The method of claim 9, wherein step b) classifies the subject by comparing the concentration obtained in a) for one or two of the microbiota-associated markers, or a combined score of the same, in the subject's biological sample, with one or more reference values; wherein said comparison between the concentration obtained in a) for the one or two of the microbiota-associated markers, or a combined score of the same, with said one or more reference values provides an indication of a risk of the subject having precancerous anal lesions consisting of high-degree squamous intraepithelial lesions (HSIL).

11. The method of claim 10, wherein said one or more reference values are about 50 ng of succinyl-CoA /mg of total protein present in the intracellular content of the biological sample and/or about 150 ng cobalamin / mg of total protein present in the intracellular content of the biological sample, and wherein the subject is classified as at risk of having precancerous anal lesions consisting of high-degree squamous intraepithelial lesions (HSIL) if:
a. the intracellular concentration of succinyl-CoA obtained in a) is greater than about 50 ng succinyl-CoA /mg of total protein present in the biological sample; and
b. the intracellular concentration of cobalamin obtained in a) is greater than about 150 ng of cobalamin /mg of total protein present in the biological sample.

12. A computer-implemented method for determining a risk of a subject having precancerous anal lesions consisting of high-degree squamous intraepithelial lesions (HSIL), comprising the following steps:
a. receiving or storing or having access to data comprising the intracellular concentration of succinyl CoA and/or cobalamin obtained from the microbial cells of a biological sample comprising anal microbial cells;
b. Calculating a score value based on the comparison of the intracellular concentration of succinyl CoA and/or cobalamin, or a combined score of the same, obtained from the microbial cells of a biological sample comprising anal microbial cells received or stored in accordance with step a) with one or more reference values; and
c. Returning as a result a risk or an indication of whether a subject has precancerous anal lesions consisting of high-degree squamous intraepithelial lesions (HSIL) based on the calculated score value.

13. A data processing apparatus/device/system comprising means for carrying out the steps a) to c) of claim 12 and preferably further comprises means for detecting and/or quantifying succinyl CoA and/or cobalamin.

14. A computer program [product] comprising instructions which, when the program is executed by a computer, cause the computer to carry out the steps of claim 12.

15. An anti-cancer therapy suitable for treating a subject having precancerous anal lesions consisting of high-degree squamous intraepithelial lesions (HSIL), wherein said subject has been classified as having or being at risk of having high-grade squamous intraepithelial lesions (HSIL) according to any of claims 9 to 12.
